# FASCICULE DE BREVET EUROPEEN

(11) **EP 0 869 805 B1**
(45) Date de publication et mention de la délivrance du brevet: **13.03.2002**
(21) Numéro de dépôt: 96944098.1
(22) Date de dépôt: 27.12.1996
(51) Int. Cl.: A61K 35/56, A61K 38/17, C07K 14/435

(54) **PROCEDE DE PREPARATION DE SUBSTANCES ACTIVES A PARTIR DE LA NACRE, PRODUITS OBTENUS, UTILES NOTAMMENT COMME MEDICAMENTS**
VERFAHREN ZUR HERSTELLUNG AKTIVER SUBSTANZEN AUS PERLMUTT, RESULTIERENDE PRODUKTE, GEEIGNET FÜR MEDIZINISCHE ANWENDUNGEN
METHOD FOR PREPARATION OF ACTIVE SUBSTANCES FROM NACRE, RESULTING PRODUCTS, USEFUL IN MEDICINAL APPLICATIONS

(30) Priorité: 28.12.1995 FR 9515650
(43) Date de publication de la demande: 14.10.1998
(73) Titulaire: CENTRE NATIONAL DE LA RECHERCHE SCIENTIFIQUE, 75016 Paris Cédex (FR)
(72) Inventeur: LOPEZ, Evelyne, F-75007 Paris (FR); GIRAUD, Michel, F-29140 Melgven (FR); BERLAND, Sophie, F-95150 Taverny (FR); MILET, Christian, F-77140 Saint-Pierre-les-Nemours (FR); GUTIERREZ, Gilles, F-69006 Lyon (FR)
(74) Mandataire: Warcoin, Jacques
(86) Numéro de dépôt international: FR9602098
(87) Numéro de publication internationale: WO9724133

(56) Documents cités:
- FR-A- 2 715 568

## Description

La présente invention se rapporte à un procédé de préparation de substances biologiquement actives à partir de la nacre et aux utilisations des produits purifiés pouvant être ainsi obtenus.

Ces produits seront notamment utiles en chirurgie osseuse et notamment en maxillo-faciale ou pour le traitement des pathologies dégénératives du cartilage, mais aussi en dermatologie.

La nacre a été proposée comme matériau de substitution et/ou de régénération osseuse, en particulier en chirurgie orthopédique ou maxillo-faciale, mais aussi pour la fabrication d'implants dentaires.

Il a en outre été démontré (Lopez et al, Tissue and Cell, 24, 667-679, 1992) que la nacre exerçait à distance des effets ostéoconducteur et ostéogène sur des cellules osseuses in vitro.

Il était donc souhaitable de pouvoir isoler les substances biologiquement actives présentes dans la nacre.

On sait que la nacre, ou aragonite chonchylifère, est une formation minéralisée biogène ; elle est constituée d'une matrice organique de substances fibreuses et non fibreuses représentant environ 1,7% de la masse totale (Taylor et al, Bulletin of the British Museum (Natural history) Zoology. **Suppl.** 3. 125pp. + 29 Planches, 1969) et de carbonate de calcium cristallisé sous forme d'aragonite. La structure de la nacre présente donc des similitudes avec celle de l'os, qui comprend une matrice organique et une phase minérale, constituée de phosphate de calcium sous forme d'hydroxyapatite. Par contre, environ 50% de la matrice organique de la nacre est soluble.

Des auteurs ont décrit l'extraction de protéines actives sur la formation de l'os, les BMP (ou Bone Morphogenetic Proteins) à partir de la fraction insoluble de la matrice de l'os (Urist et al, Proc. Natl. Acad. Sci. USA. **76**: 1828-1832, 1979). Ce procédé fait intervenir une déminéralisation acide qui entraîne des risques de dénaturation des produits d'intérêt.

Bien que la nacre ait été suggérée comme biomatériau depuis longtemps, des protéines analogues aux BMPs, par exemple, n'ont jamais été mises en évidence.

La Demanderesse a maintenant trouvé un procédé permettant de séparer des substances présentant un ensemble de propriétés biologiques particulièrement intéressantes en particulier car il ne comporte pas d'étape dénaturante.

C'est pourquoi la présente invention a pour objet un procédé de préparation de substances biologiquement actives à partir de la nacre, caractérisé en ce que :
a) on réduit de la nacre en une poudre de granulométrie inférieure à environ 200 µm (c'est-à-dire en général de 50 à 150 µm),
b) on la met en contact intime avec un solvant aqueux, éventuellement additionné de sels,
c) à la fin de la période de contact, on sépare la fraction insoluble de façon à récupérer la fraction aqueuse,
d) on concentre le solvant de la fraction aqueuse de manière à récupérer la fraction soluble ou entrainable à l'eau de la nacre qui est constituée des substances biologiquement actives, essentiellement dépourvue de constituants minéraux.

Il s'agit d'un procédé simple à mettre en oeuvre, qui est de préférence réalisé à une température comprise entre environ 4° C et 30° C ; de bons résultats sont obtenus à température ambiante.

La nacre utilisée peut être obtenue à partir de coquilles de mollusques, notamment d'huîtres telles que Pinctada maxima. On utilise la nacre brute, débarassée des autres éléments de coquille riches en calcite ; de préférence, on part de nacre blanche, sinon il faut prévoir une étape d'élimination des pigments.

Il s'agit d'une matière première aisément disponible, dont l'utilisation n'a pas d'impact négatif sur les populations naturelles ; en effet, la plupart de ces huîtres ou autres mollusques nacriers font l'objet d'une aquaculture.

De plus, un avantage supplémentaire est donné par le fait que la matière première peut être obtenue à partir de coquilles d'huîtres ayant produit des perles ; en effet, une huître perlière est éliminée du pool productif après avoir produit successivement au maximum 3 perles, alors qu'elle possède une épaisse couche de nacre de qualité excellente (grade A). La présente invention propose donc un débouché supplémentaire pour une utilisation de la nacre en aval de la perliculture.

La nacre peut être utilisée sans décontamination préalable. Les fractions biologiques purifiées, extraites de la nacre, provenant de bivalves grands filtreurs (800 l eau/jour) sont exemptes d'agents pathogènes susceptibles d'être actifs chez l'humain, ce qui constitue une part essentielle du cahier des charges d'un biomatériau implantable, dont l'innocuité, à court et à long terme est une préoccupation majeure actuellement. Cette préoccupation se justifie par le souci de pallier les pathologies graves pouvant se déclarer à la suite de l'utilisation, en chirurgie osseuse par exemple, d'os lyophilisé.

Dans un des modes de mise en oeuvre du procédé la nacre est amenée à une granulométrie comprise entre 50 et 100 µm.

Dans un autre mode de mise en oeuvre, la nacre est réduite en une poudre de granulométrie comprise entre 15 et 50 µm, ce qui permet d'améliorer le rendement, en approchant de la taille de l'unité cristalline.

Dans tous les cas, on procédera d'abord à un concassage suivi d'un meulage, un broyage simple n'étant pas adapté à la consistance du matériau de départ.

Le solvant de l'étape b) peut être choisi parmi l'eau pure, bidistillée ou apyrogène, ou additionnée de sels choisis notamment parmi NaCl ou le chlorhydrate de guanidine.

L'invention a en effet permis de mettre en évidence que les substances responsables de l'activité biologique de la nacre sont hydrophiles ou entraînables à l'eau, en particulier hydrosolubles, et peuvent être extraites dès la première étape du fractionnement, sous réserve de traiter la nacre finement broyée, ce qui en limite le coût de production.

Le produit susceptible d'être obtenu directement par le procédé défini précédemment comprend un mélange de protéines, qui présentent des activités biologiques de stimulation de la prolifération cellulaire, en particulier des ostéoblastes, des chondrocytes, des kératinocytes et des fibroblastes. Ces protéines peuvent être fibreuses ou non. L'invention concerne non seulement le produit ainsi obtenu mais les diverses substances purifiées par fractionnement à partir du produit soluble complet.

Elle comprend notamment des protéines, pouvant être assimilées à des protéines ancestrales, apparentées à la famille des BMP/TGF β (transforming growth factor).

Plus particulièrement, l'invention a pour objet une ou des protéines ostéoinductrices non fibreuses susceptibles d'être obtenues à partir de la nacre, et qui présentent les caractéristiques suivantes:
- elle sont solubles dans l'eau ou entrainables à l'eau,
- elle sont non cytotoxiques,
- elles augmentent l'activité phosphatase alcaline et la synthèse de collagène de type I par les ostéoblastes.

Elle concerne également des séquences nucléotidiques codant pour une telle protéine. Il doit être entendu que de telles protéines, obtenues d'une autre source ou par synthèse in vivo, par des organes ou tissus isolés ou par des cellules en culture sont également comprises dans l'invention, de même que les produits obtenus par synthèse chimique ou par génie génétique.

Le produit biologiquement actif peut être concentré à l'étape d) par dialyse et/ou lyophilisation.

Dans une variante du procédé, après l'étape d) on effectue une précipitation par l'éthanol et l'on récupère le culot qui constituera une sous-fraction biologiquement active, les produits restés en solution constituant une autre sous-fraction biologiquement active.

L'étape b) peut notamment être effectuée par mise en suspension de la poudre de nacre dans le solvant aqueux, sous agitation mécanique ; on peut procéder à 4° C pendant 24 heures, mais les durées et les températures peuvent être adaptées, par l'homme du métier, par exemple en fonction de la granulométrie de départ.

Dans un autre mode de réalisation, l'étape b) est effectuée par passage sous pression du solvant à travers la poudre de nacre immobilisée. Cette immobilisation peut par exemple être effectuée par une colonne, de type CLHP, éventuellement en mélange avec des substances de charge permettant un meilleure diffusion du solvant et évitant le compactage de la poudre de nacre.

Les différents produits obtenus selon l'invention sont utiles à titre d'implant ou de médicament, notamment destiné à augmenter la régénération des tissus notamment osseux, cartilagineux ou cutanés.

Les domaines d'application sont multiples et comprennent :
* Divers tissus :
   - tissus squelettiques : os, cartilages, ligaments, dents, cément, ivoire et tous les autres,
   - tissus cutanés et sous-cutanés,
   - tous les autres.
* Diverses pathologies :
   - vieillissement dégénératif, traumatique, tumoral, esthétique et handicaps,
   - infections rhumatologiques dont arthrose, polyarthrite...,
   - infections orthopédiques (traumatologiques et à visées correctrices),
   - pathologies du métabolisme calcique,
   - infections dermatologiques et dermatocosmétologiques,
   - ou autres...

Les produits selon l'invention pourront notamment être utilisés en chirurgie, en radiologie interventionnelle et dans toutes autres techniques de traitement.

Divers modes d'application pourront être envisagés par voie générale ou locale, avec visée d'effet action immédiate ou avec visée d'effet action retardée.

Les produits et protéines selon l'invention peuvent entrer dans des compositions pharmaceutiques. Celles-ci peuvent se présenter sous différentes formes telles que : solutions, suspensions, lyophilisats, poudres, gels, pâtes, colles, visqueux, non visqueux, revêtements, enduits, etc.

Elles pourront être utilisées par exemple dans les cas suivants:
* Pour des interventions sous imagerie
   - restauration osseuse locale (perte de susbtance) ou diffuse (déminéralisation) ; au niveau du rachis, des membres (os compact, os spongieux), des os plats de la face et de la boite crânienne, du squelette thoracique ou autres.
   - restauration cartilagineuse, disques intervertébraux et surfaces articulaires, suite par exemple à des destructions de type arthrosique ou traumatologique ou autres.
* Traitement des pathologies diverses des tissus squelettiques
   - dans tous les cas cités précédemment, en chirurgie classique
   - fabrication des greffons osseux "au modèle" en in vitro, par stimulation d'ostéoblastes ou de fibroblastes (ostéoinduction).
* Traitement des pathologies dermatologiques
   - par application locale
   - par intervention chirurgicale (injection, greffe ou autre...)
   - après production in vitro de greffons cutanés
* Dans les cas d'autogreffe de cellules osseuses, pour contribuer à stimuler et maintenir l'expression phénotypique de cellules humaines formatrices d'os (ostéoblastes) en culture. Ces ostéoblastes autologues sont destinés à être réimplantés sous la forme d'autogreffe.
* Utilisation "per os" , en vue de la régulation du métabolisme calcique.

Selon un aspect avantageux, la composition contient en outre un support solide biocompatible ; celui-ci sera dopé par l'addition en quantité définie, des produits biologiquement actifs. La composition pourra être utilisée comme matériau de comblement de façon à pouvoir restaurer le squelette dans les cas de perte et/ou défaut de substance osseuse qu'ils soient d'ordre traumatologique, esthétique ou pathologique.

Selon un autre aspect, la composition contient des excipients adaptés pour une administration par voie cutanée. Elle pourra induire la cicatrisation et la régénération cutanée.

L'invention se rapporte également à la production d'anticorps dirigés contre les différentes fractions protéiques biologiquement actives, ces anticorps pouvant être marqués par radioactivité, fluorescence, réactif chromogène ou enzymatiquement.

Elle concerne la production de sondes marquées ou non.

Selon un autre aspect l'invention concerne des kits de diagnostic contenant un anticorps ou une sonde tels que définis précédemment.

Les produits et protéines selon l'invention peuvent entrer dans des compositions cosméti>ques, destinées en particulier à lutter contre les effets du vieillissement.

Enfin l'invention comprend l'utilisation des substances et protéines hydrosolubles susceptibles d'être extraites de la nacre comme additifs de culture cellulaire, ainsi que des milieux de culture les contenant.

### Exemple I : Extraction

De la nacre de Pinctada maxima sous forme de fragments irréguliers (fournie par la société EVM Développement, SARL, 5, rue Capron. 75018 PARIS. SIRET 38348098500019) est lavée à l'eau courante puis par trois passages en eau distillée. Ces fragments sont mis sous forme micronisée, de granulométrie comprise entre 50 et 100 µm. La poudre obtenue est décontaminée aux rayons γ et est extraite, sans déminéralisation préalable, par agitation mécanique à 4° C pendant 24 heures, par de l'eau pure bidistillée ou de l'eau apyrogène ou une solution de NaCl à 25 g/l ou par une solution aqueuse de chlorhydrate de guanidine (pH 7,4). Une fraction soluble est obtenue après filtration sous vide, concentrée par lyophilisation puis dyalisée contre de l'eau avec une membrane de 40 mm de diamètre et de 12 000 à 14 000 Daltons de porosité. Une partie de la fraction obtenue est directement lyophilisée. L'autre partie est précipitée par l'éthanol (24 heures à 4° C).

L'ensemble des opérations effectuées est résumé sur le diagramme suivant : 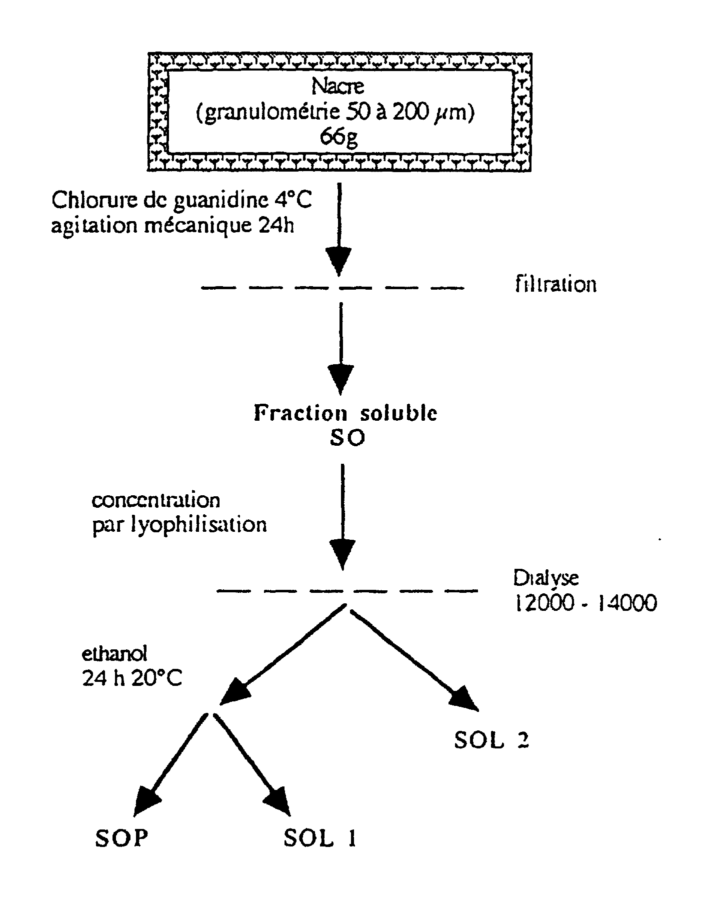
- Fractionnement, analyse des protéines et des peptides

Les spectres d'absorption en U.V. et l'analyse chromatographique des fractions brutes montrent que ces fractions sont essentiellement de nature protéique ou peptidique.

Les étapes principales du fractionnement de la matrice organique en ses divers constituants utilisent, en combinaison, les techniques classiques de chromatographie liquide haute pression (HPLC) et d'électrophorèse. L'exclusion par tailles (SEC), les échanges d'ions (IEX), les interactions hydrophobes (HIC), l'hydroxylapatite (HPHT), la chromatographie d'affinité (AFC) sont les méthodes les plus courantes de HPLC. Ces méthodes sont considérées comme ne dénaturant pas les protéines ou peptides, donc ne provoquant pas d'altération de l'activité biologique.

Les techniques électrophorétiques pour l'analyse des protéines sont l'électrophorèse capillaire de zone à haut pH (CZE), l'électrophorèse analytique et l'électrophorèse préparative. Toutes ces méthodes d'électrophorèse sont hautement résolutives et non dénaturantes.

### Exemple 2 : Tests biologiques in vitro

- Matériel biologique expérimental
   Ostéoblastes, chondroblastes, fibroblastes et kératinocytes humains en culture. Cellules de premier passage, provenant d'individus de différents sexes et d'âge variable (enfant - adulte - personne âgée).
- Critères d'évaluation utilisés
   1) Cytotoxicité, la prolifération et la migration des cellules est évaluée au moyen du test MTT, technique colorimétrique fondée sur la transformation du sel de tétrazolium (jaune) en un sel de formazan (bleu violet) par la NADPH réductase des cellules vivantes.
   2) Stimulation de l'activité des ostéoblastes maintenus à confluence, évaluée par augmentation de la présence et/ou de l'activité phosphatase alcaline.
   3) Synthèse de collagène de type I par les ostéoblastes, de type II par les chondrocytes, de type III par les fibroblastes. Ces collagènes, consituants de la matrice extracellulaire peptidique, sont des marqueurs de l'activité de ces types cellulaires.
   4) Synthèse de chondroïtine sulfate et d'acide hyaluronique, consituants de la matrice extracellulaire non peptidique sécrétée par les chondrocytes.
   5) Fixation de calcium (processus de minéralisation) sur la matrice organique extracellulaire synthétisée par les ostéoblastes (évaluée par spectrophotométrie atomique d'absorption).
   6) Synthèse de PTHrp par les kératinocytes.

A titre d'exemple :

| Activité des Fractions : test d'activité sur ostéoblastes humains en culture | | | |
|---|---|---|---|
| Extrait | MIT Différence par rapport au témoin | P. Alcaline Différence par rapport au témoin | Collagène Type I Expression |
| SOP | + | + | +++ |
| SOL 1 | + | NS | +++ |
| SOL 2 | + | + | ++ |
| NS= non significatif | | | |

| Détermination des composants de la matrice organique sur extraits : Immunodétection après électrophorèse et transfert sur membrane - sur extrait brut - | | | | | |
|---|---|---|---|---|---|
| Collagène type I | Collagène type II | Collagène type III | Décorine | PTHrp | CGrp |
| +++ | ++ | + | + | + | + |

### Exemple 3 : In vivo : critères d'évaluation utilisés

- Démonstration de l'activité des fractions : le Test de SAMPATH (SAMPATH et al., 1990), utilisé pour la caractérisation de l'activité biologique (ostéoinduction) des Bone Morphogenetic Proteins (BMP) est choisi comme modèle.

| Site sous cutané Nacre totale (Poudre) | Collagène type II ⁽¹⁾ Immunolocalisation | Collagène type I ⁽²⁾Immunolocalisation | Phosphatase alcaline ⁽³⁾ Test d'activité |
|---|---|---|---|
| 1 sem | + | - | - |
| 2 sem | + | + | + |
| 4 sem | - | + | + |

| | | | |
|---|---|---|---|
| 1) : spécifique du cartilage | | | |
| 2) : majoritaire dans l'os | | | |
| 3) : activité ostéoblastique | | | |

- Démonstration de l'activité sur la régénération cutanée : par application locale sur des lésions (scarifications, brûlures ou autres) provoquées chez l'animal (rat, lapin).

| | Lésion cutanée provoquées (rat) | |
|---|---|---|
| | témoin sham operated | + application Nacre totale (poudre) |
| Inflammation chronique | + | - |
| Cicatrisation à J+2 | +/- | +++ |

## Revendications

1. Procédé de préparation de substances biologiquement actives, **caractérisé en ce que** :
a) on réduit de la nacre en une poudre de granulométrie inférieure à environ 200 µm,
b) on la met en contact intime avec un solvant aqueux,
c) à la fin de la période de contact, on sépare la fraction insoluble de façon à récupérer la fraction aqueuse,
d) on concentre le solvant de la fraction aqueuse de manière à récupérer la fraction soluble ou entrainable à l'eau de la nacre qui est constituée des substances biologiquement actives, essentiellement dépourvues de constituants minéraux.

2. Procédé selon la revendication 1, **caractérisé en ce qu'**à l'étape a) la nacre est amenée à une granulométrie comprise entre 50 et 100 µm.

3. Procédé selon la revendication 1, **caractérisé en ce qu'**à l'étape a) la nacre est réduite en une poudre de granulométrie comprise entre 15 et 50 µm.

4. Procédé selon l'une des revendications 1 à 3, **caractérisé en ce qu'**à l'étape b), le solvant est choisi parmi l'eau pure, bidistillée ou apyrogène, ou additionnée de sels choisis notamment parmi NaCl ou le chlorhydrate de guanidine.

5. Procédé selon l'une des revendications 1 à 4, **caractérisé en ce que** l'étape d) est effectuée par concentration puis dialyse et/ou lyophilisation.

6. Procédé selon l'une des revendications 1 à 5, **caractérisé en ce qu'**après l'étape d) on effectue une précipitation par l'éthanol et l'on récupère le culot qui constituera une sous-fraction biologiquement active.

7. Procédé selon l'une des revendications 1 à 6, **caractérisé en ce que** l'étape b) est effectuée par mise en suspension de la poudre de nacre dans le solvant aqueux, sous agitation mécanique.

8. Procédé selon l'une des revendications 1 à 6, **caractérisé en ce que** l'étape b) est effectuée par passage sous pression du solvant à travers la poudre de nacre immobilisée.

9. Produits susceptibles d'être obtenus par le procédé selon l'une des revendications 1 à 8.

10. Protéines ostéoinductrices non fibreuses, analogues des BMPs ou autres, susceptibles d'être obtenues à partir de la nacre, qui présentent les caractéristiques suivantes :
- elles sont solubles dans l'eau,
- elle sont non cytotoxiques,
- elles augmentent l'activité phosphatase alcaline et la synthèse de collagène de type I par les ostéoblastes.

11. Composition pharmaceutique, **caractérisée en ce qu'**elle contient au moins un produit selon la revendication 9 ou une protéine selon la revendication 10.

12. Composition pharmaceutique selon la revendication 11, **caractérisée en ce qu'**elle contient en outre un support solide biocompatible.

13. Composition selon la revendication 11, **caractérisée en ce qu'**elle contient des excipients adaptés pour une administration par voie cutanée.

14. A titre de médicament, produits susceptibles d'être obtenus par le procédé selon l'une des revendications 1 à 8, ou protéines selon la revendication 11.

15. Utilisation d'un produit susceptible d'être obtenu par le procédé selon l'une des revendications 1 à 8 ou d'une protéine selon la revendication 10, pour la préparation d'un médicament destiné à augmenter la régénération des tissus notamment osseux, cartilagineux ou cutanés.

16. Utilisation selon la revendication 15, **caractérisée en ce que** le médicament est utile pour le traitement d'une affection choisie parmi : l'arthrose, la polyarthrite, l'ostéoporose, le vieillissement dégénératif, les pathologies tumorales et les pathologies post-traumatiques telles que pseudoarthrose et algodystrophie.

17. Composition cosmétique, **caractérisée en ce qu'**elle contient au moins un produit selon la revendication 9.

18. Milieu de culture de cellules, **caractérisé en ce qu'**il contient au moins un produit selon la revendication 9 ou une protéine selon la revendication 10.

## Patentansprüche

1. Verfahren zur Herstellung von biologisch aktiven Substanzen, **dadurch gekennzeichnet, dass** man:
a) Perlmutt in ein Pulver mit einer Teilchengröße unterhalb von etwa 200 µm zerkleinert,
b) es in innigen Kontakt mit einem wässrigen Lösungsmittel bringt,
c) am Ende des Kontaktzeitraumes die unlösliche Fraktion abtrennt, um die wässrige Fraktion zu gewinnen,
d) das Lösungsmittel der wässrigen Fraktion konzentriert, um die in Wasser lösliche oder mitführbare Fraktion des Perlmutts zu gewinnen, welche aus biologisch aktiven Substanzen zusammengesetzt ist, die im wesentlichen von mineralischen Bestandteilen befreit sind.

2. Verfahren nach Anspruch 1, **dadurch gekennzeichnet, dass** in der Stufe a) das Perlmutt auf eine Teilchengröße von 50 bis 100 µm gebracht wird.

3. Verfahren nach Anspruch 1, **dadurch gekennzeichnet, dass** im Schritt a) das Perlmutt zu einem Pulver mit einer Teilchengröße von 15 bis 50 µm zerkleinert wird.

4. Verfahren nach einem der Ansprüche 1 bis 3, **dadurch gekennzeichnet, dass** im Schritt b) das Lösungsmittel aus reinem bidestilliertem oder apyrogenem oder mit Salzen versetztem Wasser ausgewählt ist, welche insbesondere aus NaCI oder Guanidinhydrochlorid ausgewählt sind.

5. Verfahren nach einem der Ansprüche 1 bis 4, **dadurch gekennzeichnet, dass** der Schritt d) durch Konzentrieren, anschließende Dialyse und/oder Lypophilisierung bewirkt wird.

6. Verfahren nach einem der Ansprüche 1 bis 5, **dadurch gekennzeichnet, dass** man nach dem Schritt d) eine Fällung durch Ethanol vornimmt und den Bodensatz gewinnt, der eine biologisch aktive Unterfraktion bildet.

7. Verfahren nach einem der Ansprüche 1 bis 6, **dadurch gekennzeichnet, dass** der Schritt b) bewirkt wird, indem man das Perlmutt-Pulver in dem wässrigen Lösungsmittel unter mechanischem Rühren in Suspension bringt.

8. Verfahren nach einem der Ansprüche 1 bis 6, **dadurch gekennzeichnet, dass** der Schritt b) bewirkt wird, indem man das Lösungsmittel unter Druck durch das immobilisierte Perlmutt-Pulver leitet.

9. Produkte, erhältlich durch das Verfahren nach einem der Ansprüche 1 bis 8.

10. Die Knochenbildung induzierende, nicht-faserige Proteine, Analoga von BMPs oder anderen, erhältlich ausgehend von Perlmutt, welche die folgenden Eigenschaften aufweisen:
- sie sind in Wasser löslich,
- sie sind nicht zytotoxisch,
- sie erhöhen die Aktivität der alkalischen Phosphatase und die Synthese von Collagen Typ I durch die Osteoplasten.

11. Pharmazeutische Zusammensetzung, **dadurch gekennzeichnet, dass** sie mindestens ein Produkt nach Anspruch 9 oder ein Protein nach Anspruch 10 enthält.

12. Pharmazeutische Zusammensetzung nach Anspruch 11, **dadurch gekennzeichnet, dass** sie darüber hinaus einen festen biokompatiblen Träger enthält.

13. Zusammensetzung nach Anspruch 11, **dadurch gekennzeichnet, dass** sie Hilfsstoffe enthält, die an eine Verabreichung auf kutanem Weg angepasst sind.

14. Produkte, erhältlich durch das Verfahren nach einem der Ansprüche 1 bis 8 oder Proteine nach Anspruch 11 als Medikament.

15. Verwendung eines Produktes, das durch das Verfahren nach einem der Ansprüche 1 bis 8 erhältlich ist, oder eines Proteins nach Anspruch 10 für die Herstellung eines Medikaments, das dazu bestimmt ist, die Regeneration insbesondere von Knochen-, Knorpel- oder Hautgeweben zu verstärken.

16. Verwendung nach Anspruch 15, **dadurch gekennzeichnet, dass** das Medikament nützlich ist für die Behandlung einer Krankheit, die ausgewählt ist aus: Arthrose, Polyarthritis, Osteoporose, degenerativer Alterung, Geschwulstpathologien und posttraumatischen Pathologien, wie Pseudoarthrose und Algodystrophie.

17. Kosmetische Zusammensetzung, **dadurch gekennzeichnet, dass** sie mindestens ein Produkt nach Anspruch 9 enthält.

18. Zellkulturmedium, **dadurch gekennzeichnet, dass** es mindestens ein Produkt nach Anspruch 9 oder ein Protein nach Anspruch 10 enthält.

## Claims

1. Method for preparing biologically active substances, **characterized in that**:
a) nacre is reduced to a powder whose particle size is less than about 200 µm,
b) this is brought into intimate contact with an aqueous solvent,
c) at the end of the contact period, the insoluble fraction is separated so as to recover the aqueous fraction,
d) the solvent of the aqueous fraction is concentrated so as to recover the soluble or water-transportable fraction of the nacre, which consists of biologically active substances essentially free of mineral constituents.

2. Method according to Claim 1, **characterized in that**, in step a), the nacre is reduced to a particle size of between 50 and 100 µm.

3. Method according to Claim 1, **characterized in that**, in step a), the nacre is reduced to a powder with particle size between 15 and 50 µm.

4. Method according to one of Claims 1 to 3, **characterised in that**, in step b), the solvent is selected from pure, double-distilled or apyrogenic water, or water supplemented by salts selected, in particular, from NaCl or guanidine hydrochloride.

5. Method according to one of Claims 1 to 4, **characterised in that** step d) is carried out by concentration then dialysis and/or lyophilization.

6. Method according to one of Claims 1 to 5, **characterised in that**, after step d), precipitation with ethanol is carried out and the residue which will constitute a biologically active sub-fraction is recovered.

7. Method according to one of Claims 1 to 6, **characterised in that** step b) is carried out by suspending the nacre powder in the aqueous solvent under mechanical agitation.

8. Method according to one of Claims 1 to 6, **characterised in that** step b) is carried out by passing the solvent under pressure through the nacre powder which is rendered immobile.

9. Products which can be obtained by the method according to one of Claims 1 to 8.

10. Non-fibrous osteoinducing proteins, similar to BMPs or the like, which can be obtained from nacre and have the following characteristics:
- they are soluble in water,
- they are non-cytotoxic,
- they increase the alkaline phosphatase activity and the type I collagen synthesis by the osteoblasts.

11. Pharmaceutical composition, **characterised in that** it contains at least one product according to Claim 9 or one protein according to Claim 10.

12. Pharmaceutical composition according to Claim 11, **characterized in that** it furthermore contains a biocompatible solid support.

13. Composition according to Claim 11, **characterised in that** it contains excipients suitable for cutaneous administration.

14. As a medication, products which can be obtained by the method according to one of Claims 1 to 8, or proteins according to Claim 11.

15. Use of a product which can be obtained by the method according to one of Claims 1 to 8 or of a protein according to Claim 10, for the preparation of a medication intended to increase the regeneration of tissues, in particular osseous, cartilaginous or cutaneous tissues.

16. Use according to Claim 15, **characterised in that** the medication is useful for the treatment of a disorder selected from: arthrosis, polyarthritis, osteoporosis, degenerative aging, tumor pathologies and post-traumatic pathologies such as pseudoarthrosis and algodystrophy.

17. Cosmetic composition, **characterised in that** it contains at least one product according to Claim 9.

18. Cell culture medium, **characterised in that** it contains at least one product according to Claim 9 or a protein according to Claim 10.
